# EUROPEAN PATENT SPECIFICATION

(11) **EP 2 058 294 B1**
(45) Date of publication and mention of the grant of the patent: **02.01.2013**
(21) Application number: 07792980.0
(22) Date of filing: 20.08.2007
(51) Int. Cl.: C07C 69/76, C07C 67/31, C07C 67/14, C07D 313/12, C07B 61/00

(54) **METHOD FOR PRODUCING 2-(4-METHOXYCARBONYLMETHYLPHENOXYMETHYL)BENZOIC ACID METHYL ESTER**
VERFAHREN ZUR HERSTELLUNG VON 2-(4-METHOXYCARBONYLMETHYLPHENOXYMETHYL) BENZOESÄUREMETHYLESTER
PROCÉDÉ DE FABRICATION D'ESTER MÉTHYLIQUE DE L'ACIDE 2-(4-MÉTHOXYCARBONYLMÉTHYLPHÉNOXYMÉTHYL) BENZOÏQUE

(30) Priority: 21.08.2006 JP 2006224166
(43) Date of publication of application: 13.05.2009
(73) Proprietor: Sumitomo Chemical Company, Limited, Tokyo 104-8260 (JP)
(72) Inventor: KATSURA, Tadashi, Toyonaka-shi, Osaka 560-0013 (JP); HAYASHI, Taketo, Sanda-shi, Hyogo 669-1547 (JP); TANAKA, Masahide, Nishinomiya-shi, Hyogo 663-8105 (JP)
(74) Representative: Kuhnen & Wacker
(86) International application number: PCT/JP2007/066500
(87) International publication number: WO 2008/023810

(56) References cited:
- DE-A1- 2 716 230
- JP-A- 50 058 084
- US-A- 4 082 850
- US-B1- 6 222 060

## Description

### Technical Field

The present invention relates to methyl 2-(4-methoxycarbonylmethylphenoxymethyl)benzoate and a method for producing the same.

### Background of the Invention

Reaction steps represented by the following scheme are disclosed in JP-A-Hei 6-9609 for a method for producing olopatadine, which is a medicament useful as an anti-allergic drug, from dibenzoxepinacetic acid.

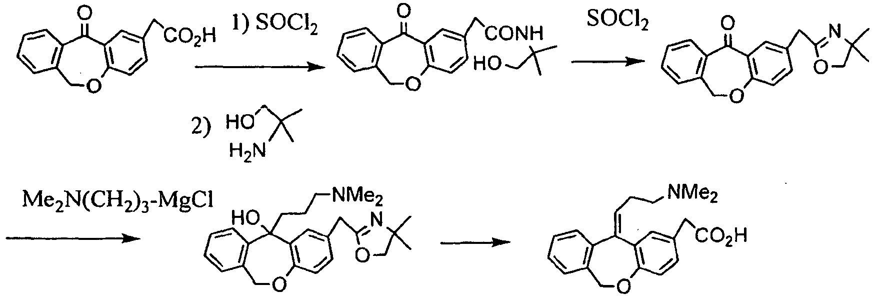

Dibenzoxepinacetic acid is produced by subjecting 2-(4-methoxycarbonylmethylphenoxymethyl)benzoic acid or its ethyl ester, which is produced from phthalide or 2-methylbenzoic acid, to the ring closing reaction. The method for the ring closing reaction is disclosed in the publication of JP-A-Hei 6-9609 and the specification of USP 4,082,850.

The method for producing the ester of 2-(4-carboxymethylphenoxymethyl)benzoic acid as described in the publication of JP-A-Hei 6-9609 is the method represented by the following scheme; phthalide at a molar amount of 1.62-fold that of methyl 4-hydroxyphenylacetate is used, for reaction at a high temperature of 150°C for 6 hours.

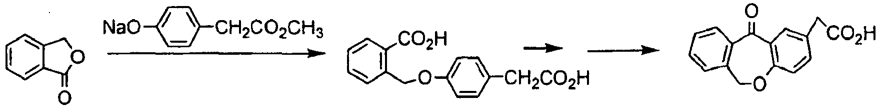

However, the method is not an economical method because the yield of 2-(4-carboxymethylphenoxymethyl)benzoic acid is not satisfactory, so that an excess amount of phthalide should be used. Since the high temperature of 150°C is required, additionally, the use of any steam line as a heat source involves difficulty, disadvantageously for industrial production.

Meanwhile, the method for producing ethyl 2-(4-ethoxycarbonylmethylphenoxymethyl)benzoate as described in the specification of USP 4,082,850 is the method represented by the following scheme, where ethyl 2-methylbenzoate is brominated with N-bromosuccinimide in carbon tetrachloride, using benzoyl peroxide as a catalyst, and then, the resulting ethyl 2-bromomethylbenzoate is allowed to react with the sodium salt of ethyl 4-hydroxyphenylacetate for 16 hours, for subsequent hydrolysis.

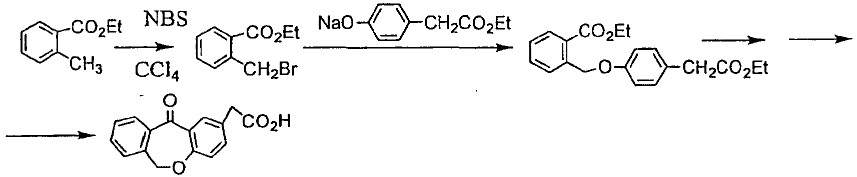

Even by the method, nonetheless, the solvent, the peroxide, the expensive halogenating agent used are limiting factors in industrial practice, problematically.

A method of synthesizing a 6,11-dihydro[b,e] oxepin-alkanoic acid is also disclosed in the US 4 082 850.

The invention relates to methods as comprised by claims 1 to 4.

A method for producing methyl 2-(4-methoxycarbonylmethylphenoxymethyl)benzoate represented by the formula (1): 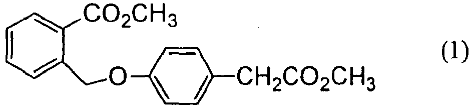 comprising a reaction of methyl 2-chloromethylbenzoate represented by the formula (2): 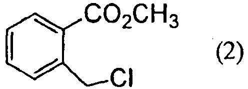 with methyl 4-hydroxyphenylacetate represented by the formula (3): 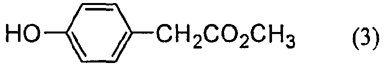 or a salt thereof.

A method for producing methyl 2-(4-methoxycarbonylmethylphenoxymethyl)benzoate comprising the following steps 1 through 3:
[Step 1]
   a step of producing 2-chloromethylbenzoyl chloride comprising a reaction of phthalide with thionyl chloride;
[Step 2]
   a step of producing methyl 2-chloromethylbenzoate represented by the formula (2): 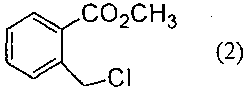 comprising a reaction of 2-chloromethylbenzoyl chloride with methanol;
[Step 3]
   a step of producing methyl 2-(4-methoxycarbonylmethylphenoxymethyl)benzoate represented by the formula (1): 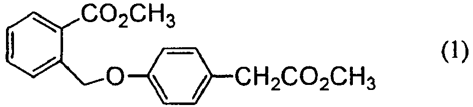 comprising a reaction of methyl 2-chloromethylbenzoate with methyl 4-hydroxyphenylacetate represented by the formula (3):

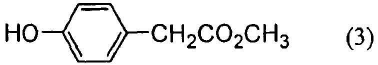

A method according to claim 3 for producing dibenzoxepinacetic acid represented by the formula (5): 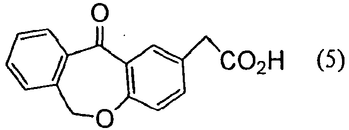 comprising hydrolyzing methyl 2-(4-methoxycarbonylmethylphenoxymethyl)benzoate to produce 2-(4-carboxymethylphenoxymethyl)benzoic acid represented by the formula (4): 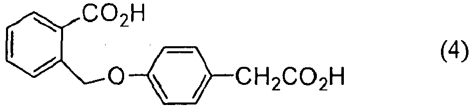 and subsequently subjecting the resulting 2-(4-carboxymethylphenoxymethyl)benzoic acid to a cyclization reaction.

In accordance with the invention, 2-(4-carboxymethylphenoxymethyl)benzoic acid can be produced efficiently at a low cost on an industrial scale. Therefore, the invention is advantageous for producing dibenzoxepinacetic acid. By the method according to the invention, a series of the reactions can be done at a temperature less than 140°C, so steam heating is readily used, advantageously from the standpoint of energy saving.

In accordance with the invention, methyl 2-(4-methoxycarbonylmethylphenoxymethyl)benzoate represented by the formula (1): 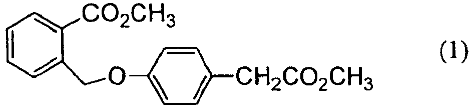 is produced by a reaction of methyl 2-chloromethylbenzoate represented by the formula (2): 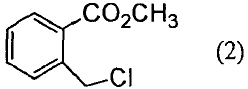 with methyl 4-hydroxyphenylacetate represented by the formula (3): 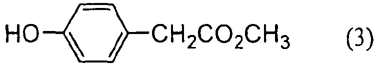 or a salt thereof.

Methyl 2-chloromethylbenzoate represented by the formula (1) can be produced for example by the steps 1 and 2 described below.

### [Step 1]

Step of producing 2-chloromethylbenzoyl chloride comprising a reaction of phthalide with thionyl chloride.

The step is carried out generally in a solvent according to the descriptions in the specification of USP 6,222,060. For example, thionyl chloride is added under heating to a solution of phthalide dissolved in an appropriate solvent, to enable the reaction of phthalide and thionyl chloride. The solvent includes for example aromatic hydrocarbons such as xylene and toluene, and halogenated hydrocarbons such as chlorobenzene. Preferably, the solvent is xylene. Additionally, an acid catalyst such as BF₃-ether complex is preferably used.

The amount of such solvent if used is preferably at a ratio of 100 to 1,000 parts by weight to 100 parts by weight of phthalide. It is preferable that a quaternary ammonium salt (for example, benzyltriethylammonium chloride, benzyltrimethylammonium chloride, and tetrabutylammonium bromide) is added to the reaction solution for promoting the reaction. The content thereof is preferably at a ratio of about 0.01 to 0.2 mole to one mole of phthalide.

When the catalyst is used, the amount thereof is preferably at a ratio of about 0.01 to 0.2 mole to one mole of phthalide.

The amount of thionyl chloride to be used is preferably at a ratio of about one to 2 moles to one mole of phthalide. The temperature of phthalide or the solution thereof is preferably 80 to 130°C just when thionyl chloride is added. Thionyl chloride is dropwise added, preferably over about 0.5 to 2 hours per one mole of thionyl chloride. After completion of the dropwise addition of thionyl chloride, the reaction mixture is preferably agitated at a temperature of about 120 to 135°C for about one to 5 hours, so as to securely promote the reaction.

After completion of the reaction, excess thionyl chloride and the solvent are removed. As the method therefor, thionyl chloride is distilled off at atmospheric pressure. Subsequently, the solvent is distilled off under reduced pressure, if necessary. In such manner, 2-chloromethylbenzoyl chloride is obtained as the resulting residue.

### [Step 2]

Step of producing methyl 2-chloromethylbenzoate represented by the formula (2): 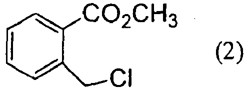 comprising a reaction of 2-chloromethylbenzoyl chloride with methanol.

At the step, 2-chloromethylbenzoyl chloride obtained at the step 1 can be used as it is without any purification for the reaction with methanol.

As the method, for example, methanol is dropwise added preferably over about 0.5 to 2 hours per one mole of 2-chloromethylbenzoyl chloride. After methanol is dropwise added to 2-chloromethylbenzoyl chloride, the resulting mixture is agitated at 30 to 60°C for about 0.5 to one hour. Methanol may be diluted with an appropriate solvent for use in the dropwise addition. Alternatively, 2-chloromethylbenzoyl chloride may be dropwise added to a mixture solution of methanol with an appropriate solvent. As the solvent, aromatic hydrocarbons such as toluene and xylene and halogenated hydrocarbons such as chlorobenzene may be listed. Preferably, toluene is used. The amount of methanol to be used is preferably at a ratio of about one to 3 moles per one mole of 2-chloromethylbenzoyl chloride.

Because hydrogen chloride generated during the reaction is contained in the reaction solution, the reaction solution is preferably neutralized with an alkali. For example, the reaction solution is dropwise added to a given amount of an aqueous potassium carbonate solution at 0 to 50°C. After neutralization, the reaction solution is washed in aqueous saline at about 15 to 35% by weight; then, the resulting organic layer is concentrated under reduced pressure, to obtain methyl 2-chloromethylbenzoate represented by the formula (2) as the residue.

Using the methyl 2-chloromethylbenzoate obtained in the aforementioned manner, then, the step 3 described below is carried out to produce methyl 2-(4-methoxycarbonylmethylphenoxymethyl)benzoate represented by the formula (1).

### [Step 3]

Step of producing methyl 2-(4-methoxycarbonylmethylphenoxymethyl)benzoate represented by the formula (1): 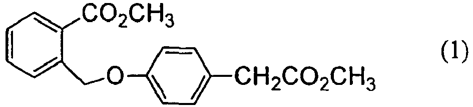 comprising a reaction of methyl 2-chloromethylbenzoate with methyl 4-hydroxyphenylacetate represented by the formula (3): 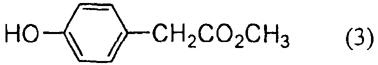 or a salt thereof.

At the step, methyl 2-chloromethylbenzoate obtained at the step 1 can be used for the next reaction, as it is without any purification. Meanwhile, methyl 4-hydroxyphenylacetate can be produced by a reaction of 4-hydroxyphenylacetic acid with methanol in the presence of an acid catalyst such as sulfuric acid, hydrochloric acid, and phosphoric acid.

The step is generally carried out in a solvent in the presence of a base under heating at about 50 to 120°C. The base to be used includes for example alkali metal carbonate salts such as potassium carbonate and sodium carbonate, and alkali metal hydroxides such as potassium hydroxide and sodium hydroxide. The solvent includes for example acid amides such as dimethylacetamide, aromatic hydrocarbons such as toluene, and halogenated hydrocarbons such as chlorobenzene.

Specifically, for example, methyl 2-chloromethylbenzoate, potassium carbonate and a solvent (for example, dimethylacetamide) are mixed together and heated to 50 to 120°C; methyl 4-hydroxyphenylacetate is added to the resulting solution, for agitation at the same temperature for about one to 12 hours. Potassium carbonate is generally used at a ratio of about 0.5 to 1.5 moles per one mole of methyl 2-chloromethylbenzoate; the solvent is generally used at a ratio of about 100 to 500 parts by weight per 100 parts by weight of methyl 4-hydroxyphenylacetate. Methyl 4-hydroxyphenylacetate is used at a molar number approximately equal to the molar number of methyl 2-chloromethylbenzoate; for example, methyl 2-chloromethylbenzoate is used at a ratio of 0.95 to 1.05 moles per one mole of methyl 4-hydroxyphenylacetate, which is dropwise added to the heated solution, for example over about 0.5 to 2 hours. Herein, methyl 4-hydroxyphenylacetate may preliminarily be allowed to react with a base for example an alkali metal carbonate salt such as potassium carbonate and sodium carbonate or an alkali metal hydroxide such as potassium hydroxide and sodium hydroxide, to produce for example the potassium salt or the sodium salt thereof. In such manner, methyl 2-chloromethylbenzoate and the salt of methyl 4-hydroxyphenylacetate can react together.

After the reaction, the objective product is separated by a general method. For example, the reaction solution is poured into water, to which an organic solvent (for example, toluene) is added to obtain an organic layer. The organic layer is washed with water, and the organic solvent is distilled off, to obtain the intended methyl 2-(4-methoxycarbonylmethylphenoxymethyl)benzoate represented by the formula (1).

By the hydrolysis of methyl 2-(4-methoxycarbonylmethylphenoxymethyl)benzoate, 2-(4-carboxymethylphenoxymethyl)benzoic acid represented by the formula (4): 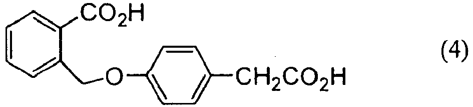 can be produced, which is then cyclized, to produce the dibenzoxepinacetic acid represented by the formula (5):

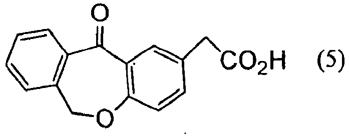

The hydrolysis of methyl 2-(4-methoxycarbonylmethylphenoxymethyl)benzoate can be done according to a general method for hydrolyzing esters with an acid or a base.

For example, methyl 2-(4-methoxycarbonylmethylphenoxymethyl)benzoate is dissolved in an alcohol solvent such as methanol and ethanol, and treated with an aqueous basic solution of potassium hydroxide and sodium hydroxide, or an aqueous acidic solution such as hydrochloric acid or sulfuric acid. The base or acid to be used is generally at a ratio of one mole to an extremely excess amount, preferably at a ratio of about one mole to 5 moles per one mole of methyl 2-(4-methoxycarbonylmethylphenoxymethyl)benzoate. The reaction temperature is generally about 20 to 80 °C.

Additionally, the cyclization reaction of 2-(4-carboxymethylphenoxymethyl)benzoic acid may be carried out under general conditions for dehydration and condensation.

By reaction of 2-(4-carboxymethylphenoxymethyl)benzoic acid with a dehydration agent such as trifluoroacetic anhydride, polyphosphoric acid, or phosphorous pentaoxide, the cyclization reaction can be progressed. In case that trifluoroacetic anhydride is used, generally, solvents such as halogenated hydrocarbons for example chlorobenzene are used, while a catalyst such as BF₃-ether complex is preferably used. The reaction temperature is generally about -20 to 50°C.

### Examples

The invention is now described more specifically in the following examples. However, the invention is never limited by the following examples.

### Example 1

Production of methyl 2-(4-methoxycarbonylmethylphenoxymethyl)benzoate by way of the production of methyl 2-chloromethylbenzoate.

### [Step 1]

In a 1-liter 4-neck flask, 643 ml of xylene, 134.1 g (1.0 mole) of phthalide, 18.2 g (0.08 mole) of benzyltriethylammonium chloride and 9.9 g (0.07 mole) of BF₃-ether complex were charged and heated to 100°C. Subsequently, 142.8 g (1.2 moles) of thionyl chloride was dropwise added to the mixture over one hour, for subsequent 2-hour agitation at 125 to 132°C. Until the inner temperature reached 135°C at atmospheric pressure, xylene and excess thionyl chloride (about 350 ml) were distilled off, and then, xylene (about 350 ml) was additionally distilled off under reduced pressure.

### [Step 2]

Eighty and one-tenth grams (80.1 g) of methanol (2.5 moles) was dropwise added to the concentrated residue obtained at the step 1 over one hour. During the term, the solution was retained at the inner temperature of 60°C or less, while the solution was cooled in a water bath. Subsequently, agitation was done at 50°C for one hour, and then, the solution was cooled to 25°C.

Then, a solution of 300 ml of toluene added to the reaction solution was dropwise added over 30 minutes to a solution of 70.0 g (0.51 mole) of potassium carbonate dissolved in 300 ml of water. The separated toluene layer was washed in 130 g of aqueous 30% saline, and concentrated under reduced pressure, to obtain 189.2 g of methyl 2-chloromethylbenzoate. The apparent yield was 102.5 %.
MS (m/z) 184 (M+)

### [Step 3]

In a 500-ml 4-neck flask, 30.4 g (0.20 mole) of 4-hydroxyphenylacetic acid and 300 ml of methanol were charged, to which 0.1 g of sulfuric acid was added, for agitation under heating and reflux for one hour. Then, methanol was mostly distilled off under reduced pressure. Subsequently, 100 ml of methanol was added to the resulting residue, from which methanol was again distilled off, to obtain methyl 4-hydroxyphenylacetate.

Thirty-eight and four-fifths grams (38.8 g) of methyl 2-chloromethylbenzoate (0.21 mole) obtained at the step 2, 30.4 g (0.22 mole) of potassium carbonate and 100 ml of dimethylacetamide were charged in a 1-liter 4-neck flask, for heating to 90 °C.

The total amount of the methyl 4-hydroxyphenylacetate obtained above was dropwise added to the solution over one hour, for subsequent agitation at 90°C for 7 hours. The reaction solution was poured into 400 ml of water, to which 200 ml of toluene was added, for separating the resulting toluene layer. The obtained toluene layer was washed in 200 ml of water, from which toluene was distilled off, to obtain 64.3 g (0.2045 mole) of methyl 2-(4-methoxycarbonylmethylphenoxymethyl)benzoate. The apparent yield was 102.2 %.
¹H NMR(400MHz, CDCl₃)
δ 3.57(s,2H), 3.68(s,3H), 3.90(s,3H), 5.49(s,2H), 6.95(d,J=8.8Hz,2H), 7.19(d,J=8.4Hz,2H), 7.37(t,J=7.6Hz,1H), 7.74(d,J=7.6,1H), 8.02(d,J=8.0,1H)

### Example 2

### Production of (11-oxo-6,11-dihydrodibenz[b,e]oxepin-2-yl)acetic acid

The total amount of methyl 2-(4-methoxycarbonylmethylphenoxymethyl)benzoate obtained in Example 1 was charged in a 4-neck flask, to which 150 ml of methanol and a solution of 17.0 g of sodium hydroxide dissolved in 100 ml of water were added, for agitation at 65°C for 2 hours.

After the reaction mixture was cooled to room temperature (about 25°C), the reaction mixture was diluted with 200 ml of water, to which 2.0 g of active charcoal was added for agitation at room temperature for one hour. Subsequently, the resulting mixture was filtered through a Buchner funnel to remove the active charcoal. The active charcoal was washed on the Buchner funnel with 100 ml of water. The filtrate was combined with the wash water, which was then heated to 60°C. A solution of 26.5g of acetic acid dissolved in 50 ml of water was dropwise added to the heated solution over 2 hours, for depositing a crystal at the same temperature. After the solution was cooled to 10°C, then, the crystal was collected by filtration with a Buchner funnel, and washed with 200 ml of water. The crystal after washing was dried under reduced pressure, to obtain 46.5 g (0.1624 mole) of 2-(4-carboxymethylphenoxymethyl)benzoic acid. The yield (the yield from 4-hydroxyphenylacetic acid in Example 1) was 81.2 %.
¹H NMR(400MHz,DMSO-d₆)
δ 3.47(s,2H), 5.45(s,2H), 6.90(d,J=8.0Hz,2H), 7.16(d,J=8.4Hz,2H), 7.47(t,J=7.6Hz,1H), 7.36(t,J=7.4Hz,1H), 7.57(d,J=7.2,1H), 7.86(d,J=7.6,1H)

The total amount of the 2-(4-carboxymethylphenoxymethyl)benzoic acid obtained above and 200 ml of chlorobenzene were charged in a 500-ml 4-neck flask, to which 75.0 g (0.3753 mole) of trifluoroacetic anhydride was added, for subsequent agitation at about 20°C for 4 hours. After adding 2.3 g (0.0162 mole) of BF₃-ether complex at -10 to 0°C over 10 minutes, agitation was done for another 30 minutes to separate the liquid layers. The resulting organic layer was washed with 200 ml of water. After the organic layer was added to a solution of 7.2 g of sodium hydroxide dissolved in 300 ml of water for agitation for 30 minutes, phase separation was done. 2.0 g of active charcoal was added to the resulting separated aqueous layer for agitation for 30 minutes; and the active charcoal was separated by filtration with a Buchner funnel. The resulting active charcoal was washed with 10 ml of water on the Buchner funnel. The filtrate and the wash water were combined together, and the resulting mixture was retained at about 40°C. To the mixture was dropwise added a mix solution of 11.3 g (0.1876 mole) of acetic acid and 50 ml of water, over 30 minutes. After completion of dropwise addition, the resulting mixture was cooled to 0 to 10°C, and filtered with a Buchner funnel to collect the deposited crystal, which was further washed with 200 ml of water. The crystal after washing was dried under reduced pressure, to obtain the entitled compound of 42.0 g. The yield [the yield from 2-(4-carboxymethylphenoxymethyl)benzoic acid] was 96.4 %, at a purity of 99.9 % as determined by HPLC.
(HPLC conditions)
Column: Inertsil ODS-5 µm (4.6 mm ID x 15 cm)
Mobile phase: 0.02 % of aqueous trifluoroacetic acid solution/acetonitrile = 5/5 → 3/7 (30 minutes)
Detection wavelength: UV 254 nm
¹H NMR(400MHz, DMSO-d₆)
δ 3.63(s,2H), 5.30(s,2H), 7.07 (d,J=8.0Hz,2H), 7.48(t,J=3.6Hz,1H), 7.55(d-d,J=7.9Hz,2H), 7.67(t,J=7.6Hz,1H), 7.78(d,J=7.6Hz,1H), 7.97(d,J=2.0Hz,1H)

### Industrial applicability

The present invention provides a method for producing methyl 2-(4-methoxycarbonylmethylphenoxymethyl)benzoate and a method for producing dibenzoxepinacetic acid. The dibenzoxepinacetic acid can be used for producing olopatadine as a pharmaceutical agent useful as an anti-allergic agent.

## Claims

1. A method for producing methyl 2-(4-methoxycarbonylmethylphenoxymethyl)benzoate represented by the formula (1): 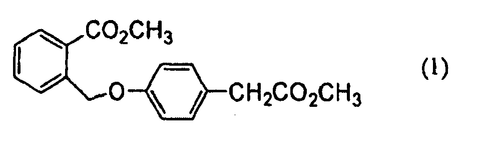 comprising a reaction of methyl 2-chloromethylbenzoate represented by the formula (2): 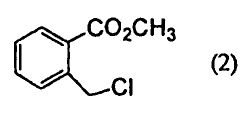 with methyl 4-hydroxyphenylacetate represented by the formula (3): 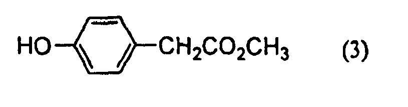 or a salt thereof.

2. The method according to claim 1 for producing methyl 2-(4-methoxycarbonylmethylphenoxymethyl)benzoate represented by the formula (1): 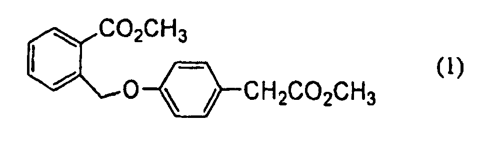 comprising the steps of:
a step of producing 2-chloromethylbenzoyl chloride comprising a reaction of phthalide with thionyl chloride;
a step of producing methyl 2-chloromethylbenzoate represented by the formula (2): 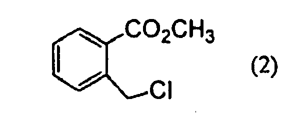
comprising a reaction of 2-chloromethylbenzoyl chloride with methanol; and a step of producing methyl 2-(4-methoxycarbonylmethylphenoxymethyl)benzoate comprising a reaction of methyl 2-chloromethylbenzoate with methyl 4-hydroxyphenylacetate represented by the formula (3):

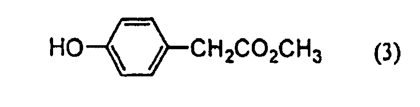

3. A method for producing dibenzoxepinacetic acid represented by the formula (5): 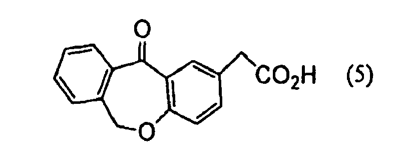 comprising a reaction of methyl 2-chloromethylbenzoate represented by the formula (2): 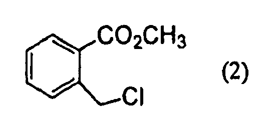 with methyl 4-hydroxyphenylacetate represented by the formula (3): 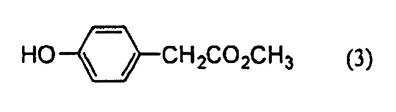 or a salt thereof to produce methyl 2-(4-methoxycarbonylmethylphenoxymethyl)benzoate represented by the formula (1): 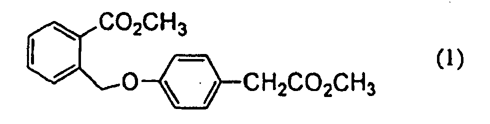 and hydrolyzing the obtained methyl 2-(4-methoxycarbonylmethylphenoxymethyl)benzoate to produce 2-(4-carboxymethylphenoxymethyl)benzoic acid represented by the formula (4): 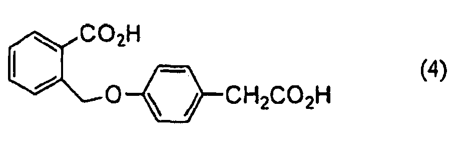 and subsequently subjecting the resulting 2-(4-carboxymethylphenoxymethyl)benzoic acid to a cyclization reaction.

4. The method according to claim 3 for producing dibenzoxepinacetic acid represented by the formula (5): 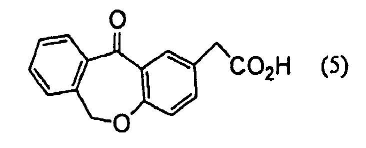 comprising the steps of:
a step of producing 2-chloromethylbenzoyl chloride comprising a reaction of phthalide with thionyl chloride;
a step of producing methyl 2-chloromethylbenzoate represented by the formula (2): 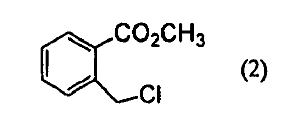
comprising a reaction of 2-chloromethylbenzoyl chloride with methanol; and a step of producing methyl 2-(4-methoxycarbonylmethylphenoxymethyl)benzoate comprising a reaction of methyl 2-chloromethylbenzoate with methyl 4-hydroxyphenylacetate represented by the formula (3): 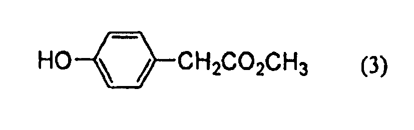
to produce methyl 2-(4-methoxycarbonylmethylphenoxymethyl)benzoate represented by the formula (1): 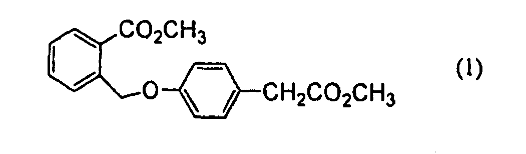
and hydrolyzing the obtained methyl 2-(4-methoxycarbonylmethylphenoxymethyl)benzoate to produce 2-(4-carboxymethylphenoxymethyl)benzoic acid represented by the formula (4): 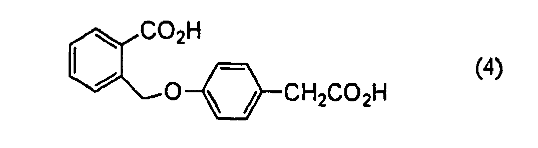
and subsequently subjecting the resulting 2-(4-carboxymethylphenoxymethyl)benzoic acid to a cyclization reaction.

## Patentansprüche

1. Verfahren zum Herstellen von Methyl-(2-(4-methoxycarbonylmethylphenoxymethyl))benzoat, das durch die Formel (1) dargstellt wird: 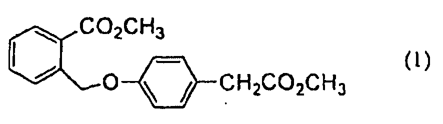 welches eine Umsetzung von Methyl-(2-Chlormethyl)benzoat, das durch die Formel (2) dargestellt wird: 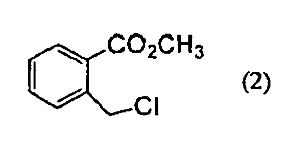 mit Methyl-4-hydroxyphenylacetat umfasst, das durch die Formel (3) dargestellt wird: 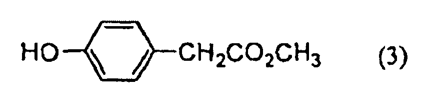 oder einem Salz desselben.

2. Verfahren nach Anspruch 1 zum Herstellen von Methyl-(2-(4-methoxycarbonylmethylphenoxymethyl))benzoat, das durch die Formel (1) dargestellt wird: 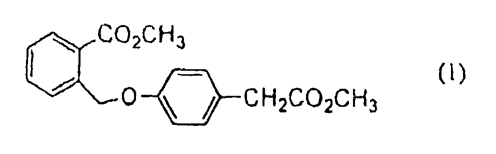 wobei das Verfahren folgende Schritte umfasst:
einen Schritt zum Herstellen von 2-Chlormethylbenzoylchlorid, der eine Umsetzung von Phthalid mit Thionylchlorid beinhaltet;
einen Schritt zum Herstellen von Methyl-(2-chlormethyl)benzoat, das durch die Formel (2) hergestellt wird: 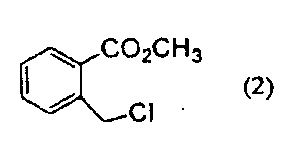
der eine Umsetzung von 2-Chlormethylbenzoylchlorid mit Methanol umfasst; und
einen Schritt zum Herstellen von Methyl-(2-(4-methoxycarbonylmethylphenoxymethyl))benzoat, der eine Umsetzung von Methyl-2-chlormethylbenzoat mit Methyl-4-hydroxyphenylacetat umfasst, das durch die Formel (3) dargestellt wird:

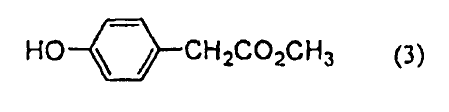

3. Verfahren zum Herstellen von Dibenzoxepinessigsäure, die durch die Formel (5) dargestellt wird: 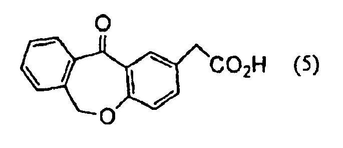 wobei das Verfahren eine Umsetzung von 2-Chlormethylbenzoat umfasst, das durch die Formel (2) dargestellt wird: 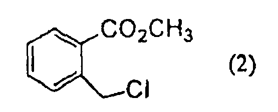 mit Methyl-4-hydroxyphenylacetat, das durch die Formel (3) dargestellt wird: 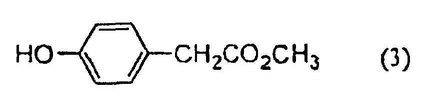 oder einem Salz desselben, um Methyl-(2-(4-methoxycarbonylmethylphenoxymethyl))benzoat herzustellen, das durch die Formel (1) dargestellt wird: 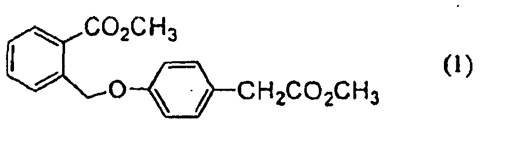 und ein Hydrolysieren des erhaltenen Methyl-(2-(4-methoxycarbonylmethylphenoxymethyl))benzoats, um eine 2-(4-Carboxymethylphenoxymethyl)benzoesäure herzustellen, die durch die Formel (4) dargestellt wird: 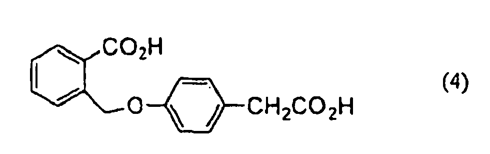 und eine anschließende Cyclisierungsreaktion der resultierenden 2-(4-Carboxymethylphenoxymethyl)benzoesäure.

4. Verfahren nach Anspruch 3 zum Herstellen einer Dibenzoxepinessigsäure, die durch die Formel (5) dargestellt wird: 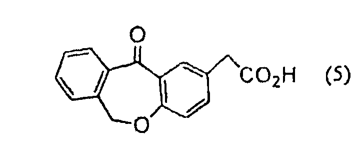 wobei das Verfahren folgende Schritte umfasst:
einen Schritt zum Herstellen eines 2-Chlormethylbenzoylchlorids, der eine Umsetzung von Phthalid mit Thionylchlorid beinhaltet;
einen Schritt zum Herstellen von Methyl-2-chlormethylbenzoat, das durch die Formel (2) dargestellt wird: 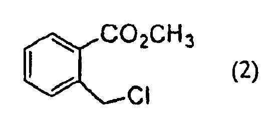
der eine Reaktion von 2-Chlormethylbenzoylchlorid mit Methanol beinhaltet; und
einem Schritt zum Herstellen von Methyl-(2-(4-methoxycarbonylmethylphenoxymethyl))benzoat, der eine Umsetzung von Methyl-2-chlormethylbenzoat mit Methyl-4-hydroxyphenylacetat beinhaltet, die durch die Formel (3) dargestellt wird: 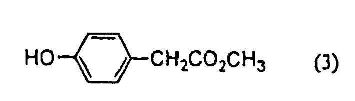
um Methyl-(2-(4-methoxycarbonylmethylphenoxymethyl))benzoat herzustellen, das durch die Formel (1) dargstellt wird: 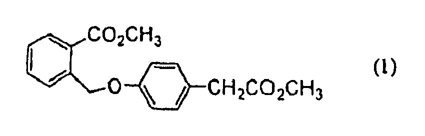
und ein Hydrolysieren des erhaltenen Methyl-(2-(4-methoxycarbonylmethylphenoxymethyl))benzoats, um 2-(4-Carboxymethylphenoxmethyl)benzoesäure herzustellen, die durch die Formel (4) dargestellt wird. 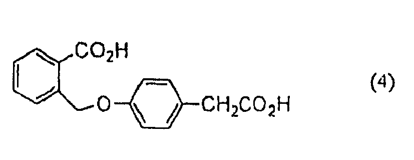
und anschließend der Unterwerfung der resultierenden 2-(4-Carboxymethylphenoxymethyl)benzoesäure einer Cyclisierungsreaktion.

## Revendications

1. Procédé pour produire le 2-(4-méthoxycarbonylméthylphénoxyméthyl)-benzoate de méthyle représenté par la formule (1) : 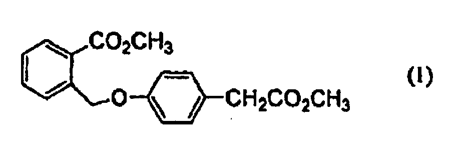 comprenant une réaction du 2-chlorométhylbenzoate de méthyle représenté par la formule (2) : 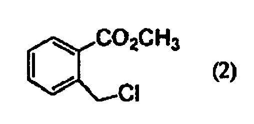 avec du 4-hydroxyphénylacétate de méthyle représenté par la formule (3) : 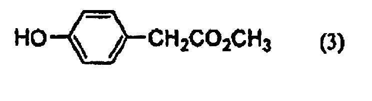 ou un sel de celui-ci.

2. Procédé selon la revendication 1 pour produire le 2-(4-méthoxy-carbonylméthylphénoxyméthyl)benzoate de méthyle représenté par la formule (1) : 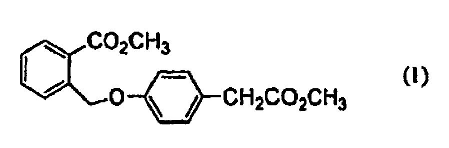 comprenant :
une étape de production du chlorure de 2-chlorométhylbenzoyle comprenant une réaction du phtalide avec du chlorure de thionyle ;
une étape de production de 2-chlorométhylbenzoate de méthyle représenté par la formule (2) : 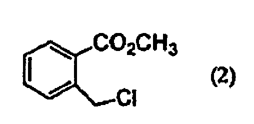
comprenant une réaction du chlorure de 2-chlorométhylbenzoyle avec du méthanol ; et
une étape de production de 2-(4-méthoxycarbonylméthylphénoxyméthyl)-benzoate de méthyle comprenant une réaction du 2-chlorométhylbenzoate de méthyle avec du 4-hydroxyphénylacétate de méthyle représenté par la formule (3) :

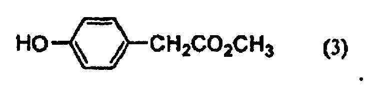

3. Procédé pour produire l'acide dibenzoxépinacétique représenté par la formule (5) : 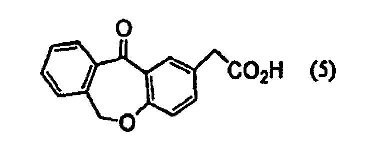 comprenant une réaction du 2-chlorométhylbenzoate de méthyle représenté par la formule (2) : 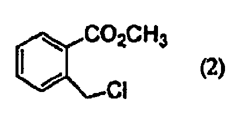 avec du 4-hydroxyphénylacétate de méthyle représenté par la formule (3) : 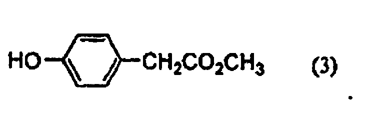 ou un sel de celui-ci pour produire le 2-(4-méthoxycarbonylméthyl-phénoxyméthyl)benzoate de méthyle représenté par la formule (1) : 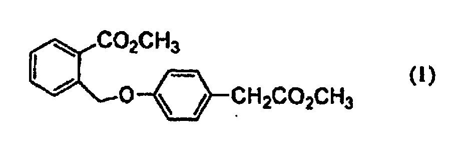 et hydrolyser le 2-(4-méthoxycarbonylméthylphénoxyméthyl)benzoate de méthyle obtenu pour produire l'acide 2-(4-carboxyméthylphénoxyméthyl)benzoïque représenté par la formule (4) : 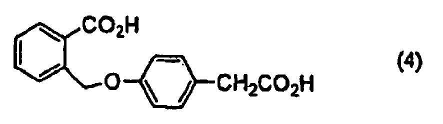 et ensuite soumettre l'acide 2-(4-carboxyméthylphénoxyméthyl)benzoïque résultant à une réaction de cyclisation.

4. Procédé selon la revendication 3, pour produire l'acide dibenzoxépinacétique représenté par la formule (5) : 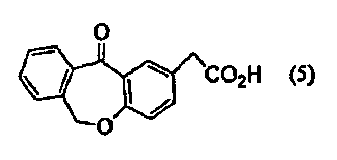 comprenant :
une étape de production du chlorure de 2-chlorométhylbenzoyle comprenant une réaction du phtalide avec du chlorure de thionyle ;
une étape de production de 2-chlorométhylbenzoate de méthyle représenté par la formule (2) : 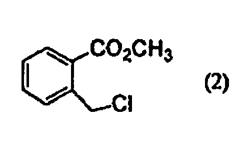
comprenant une réaction du chlorure de 2-chlorométhylbenzoyle avec du méthanol ; et
une étape de production de 2-(4-méthoxycarbonylméthylphénoxyméthyl)-benzoate de méthyle comprenant une réaction du 2-chlorométhylbenzoate de méthyle avec du 4-hydroxyphénylacétate de méthyle représenté par la formule (3) : 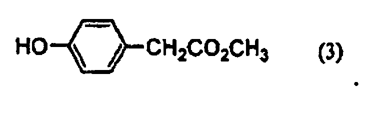
pour produire le 2-(4-méthoxycarbonylméthylphénoxyméthyl)benzoate de méthyle représenté par la formule (1) : 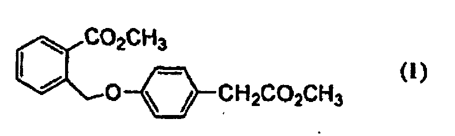
et l'hydrolyse du 2-(4-méthoxycarbonylméthyl-phénoxyméthyl)benzoate de méthyle obtenu pour produire l'acide 2-(4-carboxyméthylphénoxyméthyl)benzoïque représenté par la formule (4) : 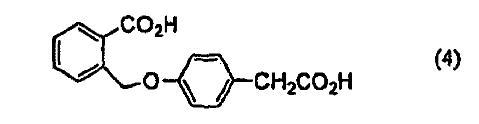
et ensuite la soumission de l'acide 2-(4-carboxyméthylphénoxyméthyl)benzoïque résultant à une réaction de cyclisation.
